(19) Europäisches Patentamt
European Patent Office
Office européen des brevets

(11) Publication number: **0 053 434**
**B1**

(12) # EUROPEAN PATENT SPECIFICATION

(45) Date of publication of patent specification: 27.08.86

(51) Int. Cl.$^4$: **C 07 C 101/18**, C 07 C 101/62, C 07 C 101/00

(21) Application number: 81305128.1

(22) Date of filing: 29.10.81

(54) **Compounds and method for treatment or prophylaxis of cardiac disorders.**

(30) Priority: 28.11.80 US 211340

(43) Date of publication of application:
09.06.82 Bulletin 82/23

(45) Publication of the grant of the patent:
27.08.86 Bulletin 86/35

(84) Designated Contracting States:
BE IT

(56) References cited:
DE-A-1 922 003

(73) Proprietor: AMERICAN HOSPITAL SUPPLY CORPORATION
One American Plaza
Evanston, IL 60201 (US)

(72) Inventor: Erhardt, Paul W.
811 Cambridge Drive
Mundelein Illinois 60060 (US)
Inventor: Borgman, Robert J.
1735 Victoria Road
Mundelein Illinois 60060 (US)
Inventor: O'Donnell, John P.
109 Williams Street
Morgantown West Virginia 26505 (US)

(74) Representative: Seaborn, George Stephen et al
c/o Edward Evans & Co. Chancery House 53-64
Chancery Lane
London WC2A 1SD (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 053 434 B1

Courier Press, Leamington Spa, England.

**Description**

Background of the invention

The present invention relates to compounds for the treatment or prophylaxis of cardiac disorders. More particularly, the invention relates to compounds which are β-adrenergic blocking agents.

The therapeutic and prophylactic uses of compounds which block sympathetic nervous stimulation of β-adrenergic receptors in the heart, lungs, vascular system and other organs are well documented. Typically, such compounds are administered therapeutically to patients suffering from ischemic heart disease or myocardial infarction for the purpose of reducing heart work, i.e., heart rate and contractile force. Reducing heart work reduces oxygen demand, and may also actually increase oxygen supply. Thus reducing heart work can aid in the prevention of further tissue damage and can relieve angina pectoris.

β-Adrenergic stimulation may also aggravate or cause arrhythmias because of increased levels of catecholamines. Thus β-blocking agents may be employed to reduce the risks of arrhythmias.

Compounds have been discovered which selectively block β-adrenergic receptors in various organs. Beta receptors in the heart are generally referred to as $\beta_1$ receptors, and those associated with vasodilation and bronchodilation are $\beta_2$ receptors. Non-selective β-blockers are not preferred for the treatment of cardiac disorders because of their hypertensive action and potention undesirable effects on asthmatic patients. A number of $\beta_1$ selective adrenergic blocking agents have been discovered. Smith, L. H., *J. Appl. Chem. Biotechnol., 28,* 201—212 (1978). Most of such compounds are structural variations of 1-amino-3-aryloxy-2-propanol.

Heretofore, the emphasis in β-blocker research has been to develop compounds which can be administered to cardiac patients over long periods of time. For example, German patent application (DE—A) 1922003 filed April 30, 1964, discloses β-blocking compounds suitable for conventional administration. However, often it is desirable in the critical care setting to quickly reduce heart work or improve rhythmicity during a cardiac crisis, e.g., during or shortly after a myocardial infarction. Conventional β-blocking agents can be employed for such treatment, but their duration of action may be much longer than desired by the physician. A β-blocking agent possessing a long duration of action does not allow precise control of heart work or prompt reversal of the β-blocking effect, which may be required in a critical care setting. For instance, if heart output becomes dangerously low, it is desirable to quickly reduce or eliminate β-blocking activity. The lingering activity of available β-blocking agents can be counterproductive and can greatly complicate the therapeutic decisions required of the physician during such critical care of cardiac patients.

Summary of the invention

In accordance with the present invention, disclosed herein is a compound for the treatment of prophylaxis of cardiac disorders in a mammal, having the formula:

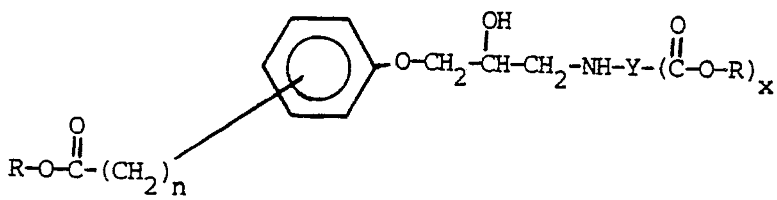

wherein Y is a straight or branched divalent or trivalent hydrocarbon group of from 1 to 10 carbon atoms or aralkyl of from 8 to 20 carbon atoms; R is lower alkyl of from 1 to 10; carbon atoms, lower alkenyl of from 2 to 10 carbon atoms, lower alkynyl of from 2 to 10 carbon atoms, aryl of from 6 to 10 carbon atoms or aralkyl, wherein the alkyl portion contains from 1 to 10 carbon atoms and the aryl portion contains from 6 to 10 carbon atoms; the

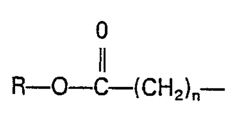

group is in the ortho or para position on the phenyl ring with respect to the amine-containing side chain; n is an integer from 0 to 2; and x is 1 or 2: or a pharmaceutically acceptable salt thereof.

Detailed description of the invention

In accordance with the present invention, it has been discovered that compounds having an ester function in external amine substituents possess β-adrenergic blocking activity and have a short duration of action. Such compounds also contain an ester-containing group on the aromatic ring. The compounds of the present invention are represented by the formula:

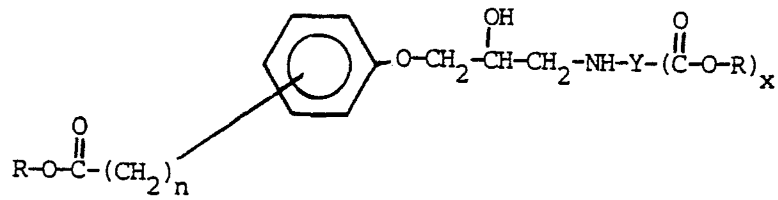

(I)

Y may be a straight or branched divalent or trivalent hydrocarbon group of from 1 to about 10 carbon atoms, e.g., methylene, ethylene, propylene, 2-ethylhexylene, 1,1-dimethyethylene, and the like or aralkyl of from 8 to about 20 carbon atoms, such as dialkylene phenyl, e.g., 4-ethylenebenzyl, 1-propylene-(4-naphthyl)-2-n-butyl, and the like.

R may be lower alkyl of from 1 to about 10 carbon atoms, such as methyl, propyl, t-butyl, 3-propylheptyl, and the like; lower alkenyl of from 2 to about 10 carbon atoms, such as ethenyl, propenyl, 4-ethyl-2-hexenyl, and the like, lower alkynyl of from 2 to about 10 carbon atoms, such as ethynyl, propynyl, 4-ethyl-3-octynyl, and the like; aryl of from 6 to about 10 carbon atoms such as phenyl, 2-tolyl, 2-methoxyphenyl, naphthyl, and the like or aralkyl, wherein the alkyl portion contains from 1 to about 10 carbon atoms and the aryl portion contains from 6 to about 10 carbon atoms, such as benzyl, phenethyl, 1-naphthylpropyl, 3,4-dimethoxyphenethyl, and the like. When two or more groups of the same designation occur in the same formula, those groups are not necessarily identical. The compounds described herein are not limited to any particular stereoisomeric configuration.

In preferred compounds, Y is a straight or branched carbon chain of from 1 to about 6 carbon atoms or aralkyl of from 8 to 12 carbon atoms. Most preferably, Y is a straight or branched carbon chain of from 1 to about 4 carbon atoms. At least one R is preferably lower alkyl of from 1 to about 5 carbon atoms, lower alkenyl of from 2 to about 5 carbon atoms, lower alkynyl of from 2 to about 5 carbon atoms, aryl of from 6 to about 8 carbon atoms, or aralkyl, wherein the alkyl portion contains from 1 to about 5 carbon atoms and the aryl portion contains from 6 to 10 carbon atoms. Most preferably, at least one R is lower alkyl of from 1 to about 4 carbon atoms or aralkyl, wherein the alkyl portion contains from 1 to about 4 carbon atoms and the aryl portion contains from 6 to about 8 carbon atoms. Particularly preferred R groups are methyl and ethyl. The integer x is preferably 1 or 2; most preferably 1; and n is 1 or 2.

Multiple ester compounds of the formula (I) are particularly short acting *in vivo.* In more preferred compounds of the formula (I), Y is a straight or branched carbon chain of from 1 to 6 carbon atoms or aralkyl of from 8 to 12 carbon atoms, and at least one R is lower alkyl of from 1 to 5 carbon atoms, lower alkenyl of from 2 to 5 carbon atoms or lower alkynyl of from 2 to 5 carbon atoms. In yet more preferred compounds of the formula (I), Y is a straight or branched carbon chain of from 1 to 4 carbon atoms, and at least one R is lower alkyl of from 1 to 4 carbon atoms. In even more preferred compounds of the formula (I), Y is methylene or ethylene, and at least one R is methyl or ethyl. In the most preferred compound of the formula (1), the

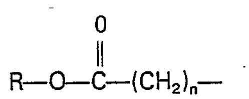

group is in the ortho position on the phenyl ring with respect to the amine-containing side chain, and n is 0.

The compounds of this invention may be administered as their pharmaceutically acceptable acid addition salts, e.g., as the hydrochloride, sulfate, phosphate, gluconate, tartrate, etc.

The compounds of the present invention may be prepared by a number of reaction procedures. The following four reaction schemes have been employed. In all of the reaction schemes, an aryl ether epoxide is used as the starting material. The aryl ether epoxide is prepared from an appropriately derivatized aryl hydroxy compound as follows:

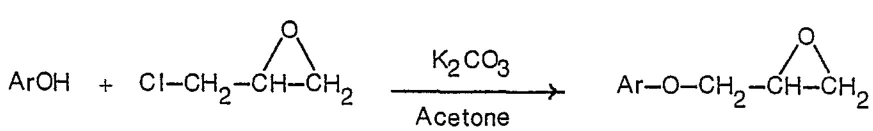


The aryl ether epoxide may then be reacted in the following manner to provide the desired product:

SCHEME I

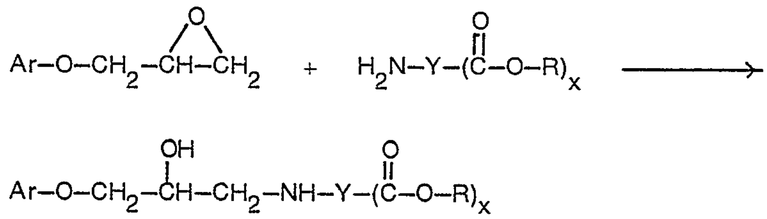

SCHEME II

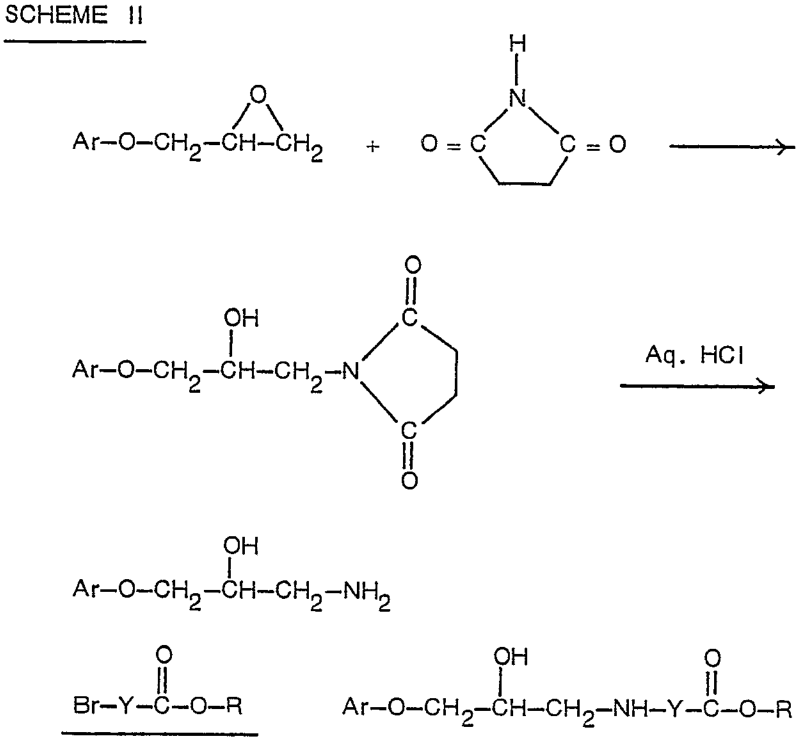


SCHEME III

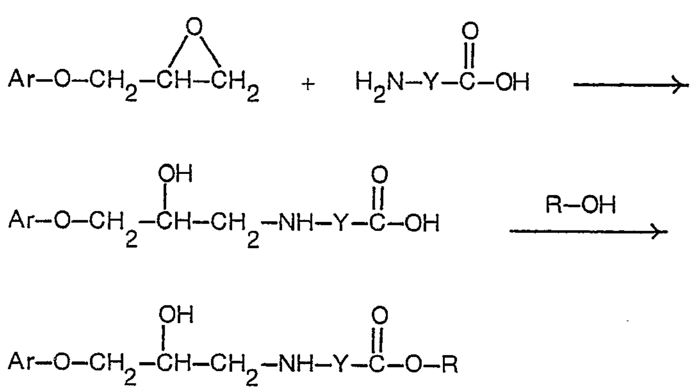

SCHEME IV

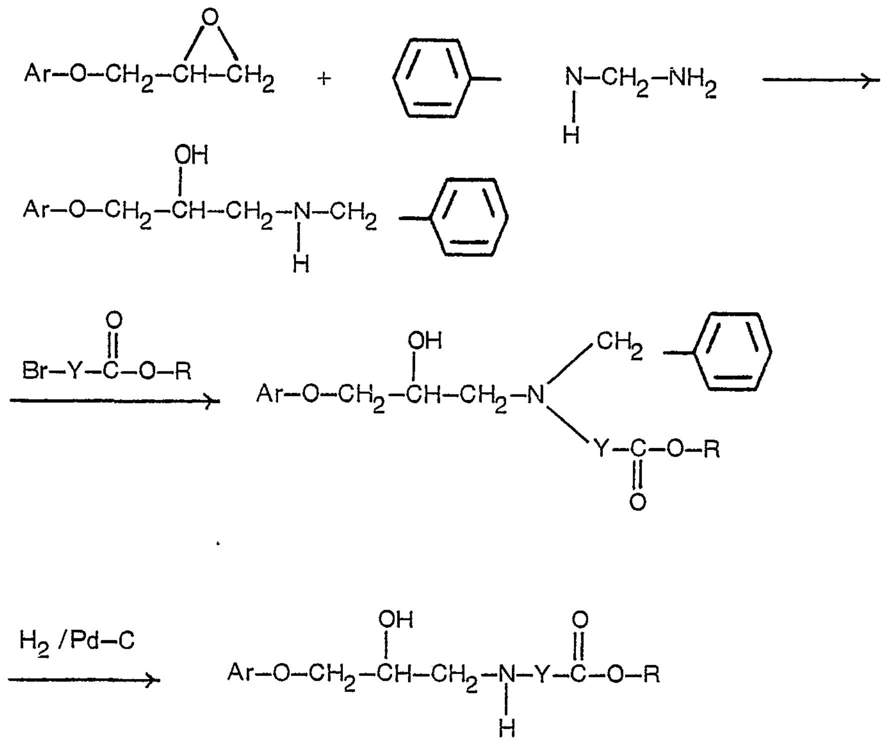


The compounds of this invention are advantageously administered parenterally, e.g., by intravenous injection or intravenous infusion. Formulations for intravenous injection preferably include the active compound as a soluble acid addition salt in a properly buffered isotonic solution.

The dosage administered to a patient and the duration of infusion will depend upon the patient's needs and the particular compounds employed. For short periods of infusion, e.g., less than about three hours, the duration of effect is thought to be determined by both metabolic effects and the distributuion phenomena. For relatively long periods of infusion, e.g., greater than about three hours, the duration of effect is thought to depend largely on metabolic effects. Accordingly, although the present methods and compounds are generally useful for short term infusion therapy, certain compounds are preferred for longer duration of infusion. The compounds have been found to be generally non-toxic within conventional dosage ranges. Dosages of from about 0.001 to about 100 mg. per kg. of body weight per hour are generally employed with preferred dosages ranging from about 0.01 to about 10 mg. per kg. of body weight per hour.

The compounds of the present invention have a relatively short duration of action compared to conventional β-blockers. *In vitro* studies in human whole blood indicate that the ester functions are subject to enzymatic cleavage. Compounds of the present invention in which the aromatic portion, Ar, is substituted with an ester-containing group, have two or more potentially labile sites for enzymatic hydrolysis. Thus β-blocking activity can be carefully controlled by regulating dosage size and rate of administration. The time required for substantially complete disappearance of the β-blocking effects of the compounds of the present invention ranges from about 5—10 minutes to about 1 hour or more. Generally, it is preferred that the recovery is accomplished within about 10—15 minutes. A short acting β-blocker can advantageously be infused at a rate sufficient to provide the desired action, e.g., titrated to the specific patient's needs, and such action can be promptly discontinued by stopping the infusion. Thus, the method of the present invention provides a very useful therapeutic alternative in the treatment or prophylaxis of cardiac disorders.

The present invention is further illustrated by the following examples which are not intended to be limiting.

Example I

This example described procedures for preparing a compound of the formula

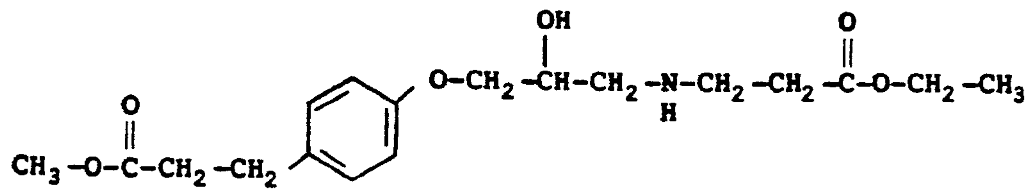

Methyl 3(4-Hydroxyphenyl)propionate

A solution of 300 gm (1.81 mole) of 3-(4-hydroxyphenyl)-propionic acid in 1 liter of anhydrous methanol containing 10 drops of concentrated $H_2SO_4$ was heated to reflux for 72 hours in a Soxhlet Extractor charged with 200 gm of 3A molecular sieves (Linde 3A, 1/16 pellets). The reaction medium was then evaporated under reduced pressure and the resulting oil taken up in 750 ml of toluene and washed with three 500 ml portions of water. The toluene phase was then dried with $MgSO_4$ and evaporated under reduced pressure to provide 228.4 gm (70%) of a clear oil which was characterized by NMR spectroscopy and utilized directly in the next step without additional purification.

Methyl 3[4-(2,3-Epoxypropoxy)phenyl]propionate

A mixture of 228 gm (1.27 mole) of methyl 3-(4-hydroxyphenyl)-propionate, 263 gm (1.90 mole) of $K_2CO_3$ and 298 ml (3.80 mole) of epichlorohydrin in 2 liters of acetone was stirred and heated to reflux for 20 hours. The reaction medium was then filtered and evaporated under reduced pressure. The resulting oil was taken up in 1 liter of toluene and washed consecutively with 500 ml water, 2 × 500 ml 1N NaOH and 2 × 500 ml water. The toluene phase was then dried over $MgSO_4$ and evaporated under reduced pressure to provide a clear oil which was further purified by vacuum distillation. The final yield of purified oil was 131.2 gm (44%): bp 156° (p=0.4 mm Hg). The NMR and IR spectra of the product were consistent with the assigned structure and the elemental analysis was consistent with the formula $C_{13}H_{16}O_4$.

Ethyl 3-[N-[2-Hydroxy-3-[4-[2-(methoxycarbonyl)ethyl]phenoxy]propyl]amino]propionate Hydrochloride

A mixture of 5 gm (0.02 mole of methyl 3-[4-(2,3-epoxy-propoxy)-phenyl]propionate, 3 gm (0.02 mole) of ethyl 3-amino-propionate hydrochloride and 2.8 ml (0.02 mole of triethylamine in 25 ml of isopropanol was heated to reflux for 4 hours. The reaction medium was then cooled and the triethylamine hydrochloride side-product which crystallized was removed by filtration. The mother liquor was then evaporated under reduced pressure and the resulting residue taken up in ethanol and treated with ethereal HC1 to provide 1 gm (12%) as a crystalline solid: mp 110—111°. The NMR spectrum was consistent with the assigned structure and the elemental analysis was consistent with the formula $C_{18}H_{28}NO_6C1$.

Example II

This example describes procedures for the preparation of a compound of the formula

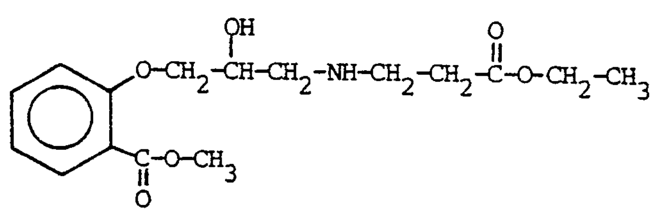

Methyl 2-(2,3-Epoxypropoxy)benzoate

The procedure of Example I for producing methyl 3-[4-(2,3-epoxypropoxy)phenyl]propionate was repeated in all essential details, except methyl salicylate was substituted for methyl 3-(4-hydroxyphenyl)propionate. The boiling point of the product was 148° (p=75μ). The NMR spectrum was consistent with the assigned structure and the elemental analysis with the formula $C_{11}H_{12}O_4$.

Ethyl 3-[N-[2-Hydroxy-3-[2-(methoxycarbonyl)phenoxy]propyl]amino]propionate oxalate hemihydrate

The procedure for Example I for producing ethyl 3-N-[2-hydroxy-3-[4-[2-(methoxycarbonyl)ethyl]phenoxy]propyl]amino]propionate hydrochloride was repeated in all essential details except methyl 2-(2,3-epoxypropoxy) benzoate was substituted for 3[4-(2,3-epoxypropoxy)phenyl]propionate and the product was crystallized as its oxlate salt from 2-propanol ether and then recrystallized from acetone. Approximately 2 gm (25%) of product was obtained having a melting point of 90—91°C. The NMR spectrum was consistent with the assigned structure and the elemental analysis was consistent with the formula $C_{18}H_{25}NO_{10}.1/2H_2O$.

Example III

This example describes procedures for the preparation of a compound of the formula:

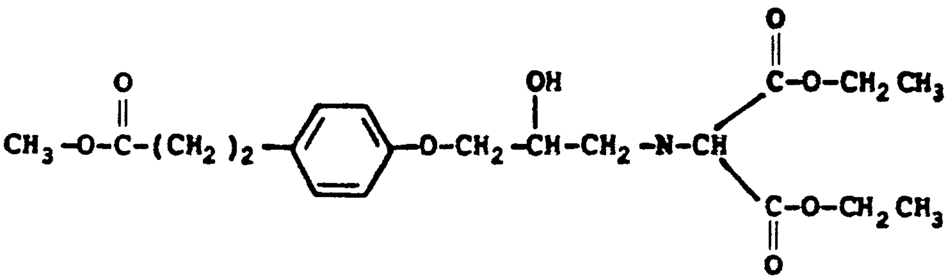

Diethyl 2-Amino-[N-[2-hydroxy-3-(4-(2-carbomethoxyethyl)-phenoxypropyl]]propanedioate Hydrochloride

A mixture of 11.8 gm (0.05 mole) of methyl 3-[4-(2,3-epoxypropoxy)phenyl]propionate, 10.6 gm (0.05 mole) of diethyl 2-aminomalonate hydrochloride and 7 ml (0.05 mole) of triethylamine in 100 ml of isopropanol was heated to reflux for 4 hours. The reaction medium was then filtered and evaporated under vacuum. The resulting oil was taken up in 100 ml toluene and washed with 3 × 50 ml water. The organic phase was then dried over $MgSO_4$ and evaporated under vacuum. The free amine oil was taken up in ethanol and converted to its hydrochloride salt by adding ethereal HCl. Approximately 4 gm (20%) of white crystals were obtained: mp 144—145°. The NMR spectrum was consistent with the assigned structure and the elemental analysis was consistent with the formula $C_{20}H_{30}NO_8C1$.

Examples IV and V

Two of the compounds of the present invention were tested for β-blocking activity *in vitro* using guinea pig right atria and guinea pig tracheal strips mounted in a tissue bath containing oxgenated (95% $O_2$—5% $CO_2$) Krebs physiological salt solution of 37°C. Each tissue was suspended between a fixed glass rod and a Statham Universal Transducer connected to a Beckman recorder. Atria were allowed to beat spontaneously under a loading tension of approximately 0.5 g. Intrinsic depressant or stimulant activity was determined for each compound by progressively increasing concentrations in the tissue baths at 60-minute intervals. Tissues were not washed between increments. The maximum concentration showing little or no cardiodepressant activity was chosen for blockade experiments. Changes in rate in response to isoproterenol were measured in the absence and presence of test compounds. Spiral strips of guinea pig trachea were suspended under 5 gm resting tension and incubated with phentolamine, tropolone and cocaine. Active tension was generated by addition of carbachol (3.0 × $10^{-7}$M) and decreased in tension in response to isoproterenol were quantitated. Cumulative concentration-response curves were produced with isoproterenol both before and after 60-minute incubation of test compounds with atria and trachea. The blocking potency of test compounds was estimated by computing $pA_2$ values ($-\log K_B$) by the method of Furchgott, The Pharmacological Differentiation of Adrenergic Receptors, *Ann. N.Y. Acad. Sci.; 139:* 553—570 (1967). Comparison of blockade of right atrial and tracheal responses to isoproterenol permitted assessment of cardioselectivity of test compounds; i.e., cardioselective compounds are relatively more effective in blocking atrail rate than tracheal force responses to isoproterenol. The degree of cardioselectivity was estimated from the ratio, $K_B$ tracheal/$K_B$ atrial (10($pA_2$atria—$pA_2$trachea)). A ratio greater than one indicates cardioselectivity. Test drugs were dissolved in distilled water and added to the bath (30 ml) in a volume of 10 or 100 ul. The results of the *in vitro* tests are contained in Table I. All of the test compounds were active β-blockers.

Example VI

The duration of β-blockade was determined *in vivo* using pentobartital-anesthetized dogs instrumented for measurement of heart rate using a Beckman cardiotachometer triggered electronically by a phasic aortic blood pressure signal. Both vagus nerves were severed in the cervical region and the animals were mechanically ventilated. The test employed a 3-hour infusion of test compound. Bolus doses of isoproterenol (0.4 ug/kg) were used to assess the degree of β-blockade and recovery from β-blockade after termination of the infusion. The doses were spaced at 10-minute intervals and were given before, during and following the infusion of test compounds. The infusion rate was adjusted so that at the end of the 3-hour infusion period the degree of isoproterenol inhibition averaged about 50% of control. The results of isoproterenol inhibition averaged about 50% of control. The results of the 3-hour infusion are shown in Table II, comparing the duration of action of the compound described in Example II to those of the conventional β-blockers, propranolol and practolol.

TABLE I

| Example | Test Compound (Numerical designation indicates previous example which describes preparation of compound) | $pA_2$ | | Cardioselectivity |
|---|---|---|---|---|
| | | Atria | Trachea | |
| IV | I | 5.9 | < 5.0 | > 8 |
| V | II | 6.6 | 6.6 | 1.0 |
| Propranolol | | 8.7 | 8.9 | 0.6 |
| Practolol | | 6.6 | 5.8 | 6.0 |

TABLE II

| Example | Test Compound (Numerical designation indicates previous example which describes preparation of compound) | Potency (mg/kg/180 min) | %I* | 80% Recovery Time (Minutes) | No. of Experiments |
|---|---|---|---|---|---|
| VI | II | 27.4 ± 6.0 | 53 ± 2 | 10 ± 2 | 5 |
| Propanolol | | | | No appreciable recovery after several hours | |
| Practolol | | | | No appreciable recovery after several hours | |

* Percent inhibition of heart rate response to isoproterenol

**Claims**

1. A compound of the formula

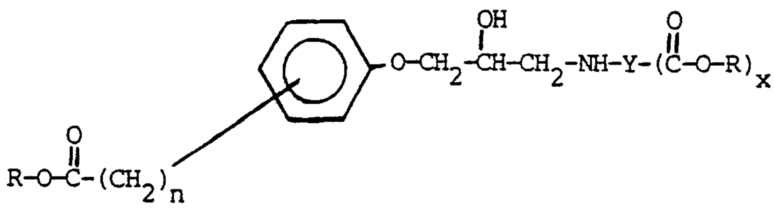

wherein Y is a straight or branched divalent or trivalent hydrocarbon group of from 1 to 10 carbon atoms or aralkyl of from 8 to 20 carbon atoms; each R is lower alkyl of from 1 to 10 carbon atoms, lower alkenyl of from 2 to 10 carbon atoms, lower alkynyl of from 2 to 10 carbon atoms, aryl of from 6 to 10 carbons atoms or aralkyl, wherein the alkyl portion contains from 1 to 10 carbon atoms and the aryl portion contains from 6 to 10 carbon atoms; the

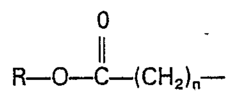

group is in the ortho or para position on the phenyl ring with respect to the amine-containing side chain; n is an integer from 0 to 2; and x is 1 or 2; or a pharmaceutically acceptable salt thereof.

2. The compound of claim 1, wherein Y is a straight or branched carbon chain of from 1 to 6 carbon atoms or aralkyl of from 8 to 12 carbon atoms; and at least one R is a lower alkyl of from 1 to 5 carbon atoms, lower alkenyl of from 2 to 5 carbon atoms, lower alkynyl of from 2 to 5 carbon atoms; or a pharmaceutically acceptable salt thereof.

3. The compound of claim 1, wherein Y is a straight or branched carbon chain of from 1 to 4 carbon atoms; and at least one R is lower alkyl of from 1 to 4 carbon atoms; or a pharmaceutically acceptable salt thereof.

4. The compound of claim 1, wherein Y is methylene or ethylene and at least one R is methyl or ethyl; or a pharmaceutically acceptable salt thereof.

5. The compound of claim 4, wherein the

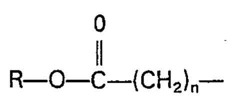

group is in the ortho position on the phenyl ring with respect to the amine-containing side chain, and n is 0.

6. The compound of the formula

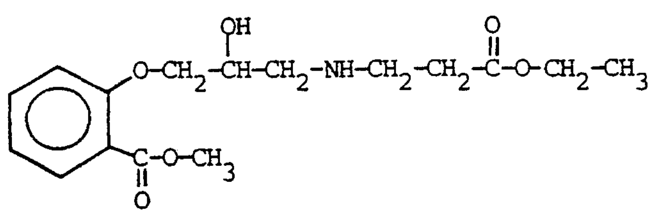

or a pharmaceutically acceptable salt thereof.

7. The compound of claim 1, 2, 3, 4, 5 or 6 as the hydrochloride, sulfate, phosphate, gluconate or tartrate acid addition salt.

8. The pharmaceutical composition comprising a β-blocking effective amount of a β-blocking ester compound of the formula

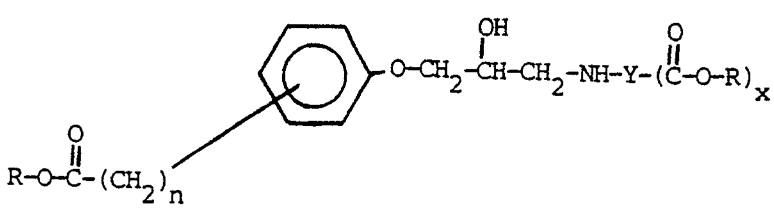

wherein Y is a straight or branched divalent or trivalent hydrocarbon group of from 1 to 10 carbon atoms or aralkyl of from 8 to 20 carbon atoms; each R is lower alkyl of from 1 to 10 carbon atoms; lower alkenyl of from 2 to 10 carbon atoms, lower alkynyl of from 2 to 10 carbon atoms, aryl of from 6 to 10 carbon atoms or aralkyl, wherein the alkyl portion contains from 1 to 10 carbon atoms and the aryl portion contains from 6 to 10 carbon atoms; the

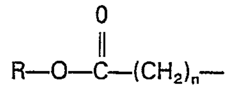

group is in the ortho or para position on the phenyl ring with respect to the amine-containing side chain; n is an integer from 0 to 2; and x is 1 or 2; or a pharmaceutically acceptable salt thereof; said compound being dissolved in a properly buffered isotonic solution suitable for intravenous administration.

9. The composition of claim 8, wherein Y is a straight or branched carbon chain of from 1 to 6 carbon atoms or aralkyl of from 8 to 12 carbon atoms; and at least one R is lower alkyl of from 1 to 5 carbon atoms, lower alkenyl of from 2 to 5 carbon atoms, lower alkynyl of from 2 to 5 carbon atoms; or a pharmaceutically acceptable salt thereof.

10. The composition of claim 8, wherein Y is a straight or branched carbon chain of from 1 to 4 carbon atoms; and at least one R is lower alkyl of from 1 to 4 carbon atoms; or a pharmaceutically acceptable salt thereof.

11. The composition of claim 8, wherein Y is a methylene or ethylene and at least one R is methyl or ethyl; or a pharmaceutically acceptable salt thereof.

12. The composition of claim 11, wherein the

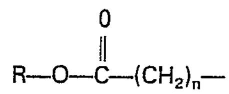

group is in the ortho position on the phenyl ring with respect to the amine-containing side chain, and n is 0.

13. A pharmaceutical composition comprising a β-blocking effective amount of a β-blocking ester compound of the formula

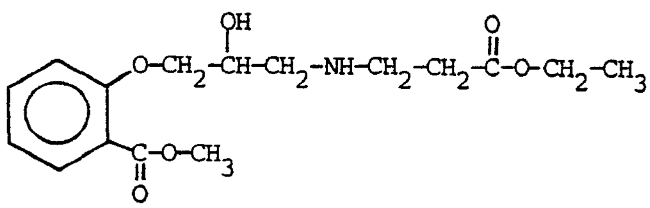

or a pharmaceutically acceptable salt thereof; said compound being dissolved in a properly buffered isotonic solution suitable for intravenous infusion.

14. The composition of claim 8, 9, 10, 11, 12 or 13 wherein the β-blocking compound is a hydrochloride, sulfate, phosphate, gluconate or tartrate acid addition salt.

15. A compound of the formula

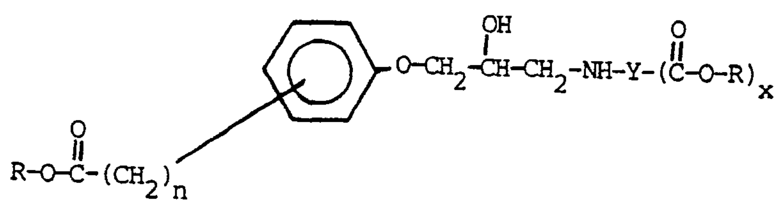

wherein Y is a straight or a branched divalent or a trivalent hydrocarbon group of from 1 to 10 carbon atoms or aralkyl of from 8 to 20 carbon atoms; each R is lower alkyl of from 1 to 10 carbon atoms, lower alkenyl of from 2 to 10 carbon atoms, lower alkynyl of from 2 to 10 carbon atoms, aryl of from 6 to 10 carbon atoms or aralkyl, wherein the alkyl portion contains from 1 to 10 carbon atoms and the aryl portion contains from 6 to 10 carbon atoms; and

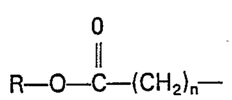

group is in the ortho or para position on the phenyl ring with respect to the amine-containing side chain; n is an integer from 0 to 2; and x is 1 or 2 or a pharmaceutically acceptable salt thereof for use as a β-blocking compound.

16. The compound of claim 15, wherein Y is a straight or branched carbon chain of from 1 to 6 carbon atoms or aralkyl of from 8 to 12 carbon atoms; at least one R is lower alkyl of from 1 to 5 carbon atoms, lower alkenyl of from 2 to 5 carbon atoms, lower alkynyl of from 2 to 5 carbon atoms; or a pharmaceutically acceptable salt thereof for use as a β-blocking compound.

17. The compound of claim 15, wherein Y is a straight or branched carbon chain of from 1 to 4 carbon atoms; at least one R is a lower alkyl of from 1 to 4 carbon atoms or a pharmaceutically acceptable salt thereof for use as a β-blocking compound.

18. The compound of claim 15, wherein Y is methylene or ethylene and R is methyl or ethyl; or a pharmaceutically acceptable salt thereof for use as a β-blocking compound.

19. The compound of claim 18 wherein the

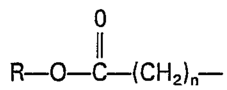

group is in the ortho position on the phenyl ring with respect to the amine-containing side chain and n is 0 for use as a β-blocking compound.

20. The compound of the formula

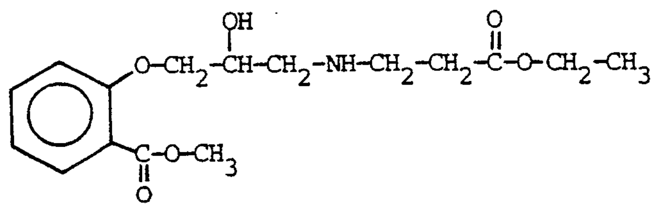

or a pharmaceutically acceptable salt thereof for use as a β-blocking compound.

21. The compound of claim 15, 16, 17, 18, 19 or 20 as the hydrochloride, sulfate, phosphate, gluconate or tartrate acid addition salt for use as a β-blocking compound.

22. A process for preparing a compound of the formula

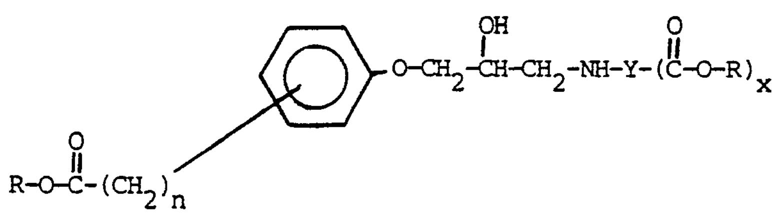

wherein Y is a straight or a branched or aralkyl of from 8 to 20 carbon atoms divalent or trivalent hydrocarbon group of from 1 to 10 carbon atoms; R is a lower alkyl of from 1 to 10 carbon atoms, lower alkenyl of from 2 to 10 carbon atoms, lower alkynyl of from 2 to 10 carbon atoms, aryl of from 6 to 10 carbon atoms or aralkyl, wherein the alkyl portion contains from 1 to 10 carbon atoms and the aryl portion contains from 6 to 10 carbon atoms; the

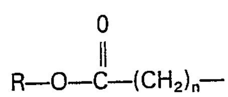

group is in the ortho or para position on the phenyl ring with respect to the amine-containing side chain; n is an integer from 0 to 2 and x is 1 or 2; or a pharmaceutically acceptable salt thereof; which comprises reacting an aryl hydrocy compound of the formula

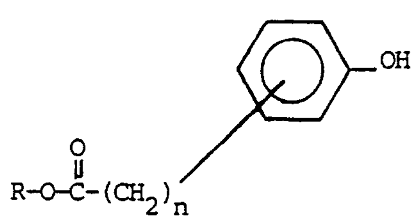

with epichlorohydrin in the presence of a suitable solvent and base at reflux temperature to give an aryl ether epoxide of the formula

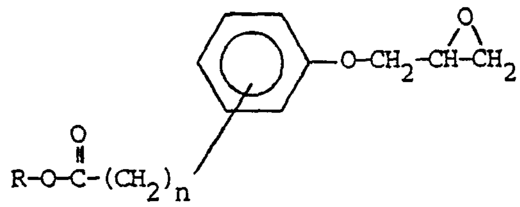

followed by reacting the aryl ether epoxide according to one of the following methods to give the final product:

(a) the aryl ether epoxide is reacted with an appropriate amine of the formula $H_2N—Y—(COOR)_x$ wherein Y and x are defined as hereinbefore, in the presence of a solvent at reflux temperature to give the final product;

(b) the aryl ether epoxide is reacted with succinimide followed by reaction of the resulting product with an appropriate acid to give an amine of the formula

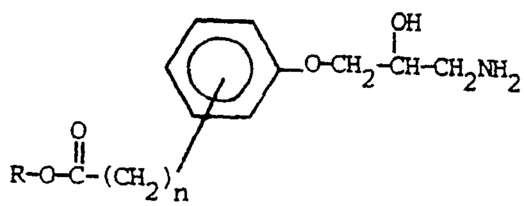

which is then reacted with a compound of the formula Br—Y—COOAR, wherein Y and R are defined as hereinbefore, to give the final product;

(c) the aryl ether epoxide is reacted with an amino acid of the formula $H_2N—Y—COOH$, wherein Y is defined as hereinbefore, followed by reaction of the product with an alcohol of the formula R—OH, wherein R is defined hereinbefore, to give the final product;

(d) the aryl ether epoxide is reacted with benzylamine by heating the reactants to give a product of the formula

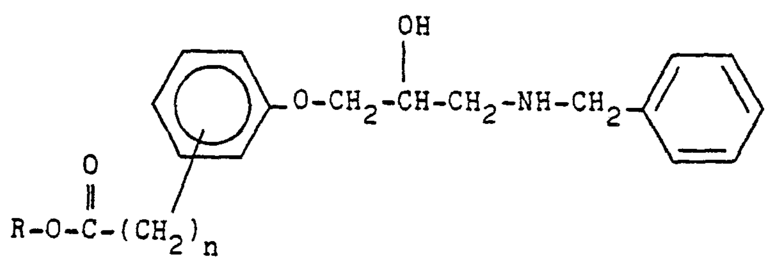 ,

which is then reacted with a compound of the formula

$$Br—Y—\overset{\overset{O}{\|}}{C}—O—R$$

followed by hydrogenation of the resulting product to remove the benzyl group and give the final product; and optionally converting the final product (a), (b), (c) or (d) to a pharmaceutically acceptable salt by reaction with an appropriate acid.

23. The process of claim 22, wherein Y is a straight or branched carbon chain of from 1 to 6 carbon atoms or aralkyl of from 8 to 12 carbon atoms; and at least one R is lower alkyl of from 1 to about 5 carbon atoms, lower alkenyl of from 2 to about 5 carbon atoms or lower alkynyl of from 2 to 5 carbon atoms.

24. The process of claim 22, wherein Y is a straight or branched carbon chain of from 1 to 4 carbon atoms; and at least one R is lower alkyl of from 1 to 4 carbon atoms.

25. The process of claim 22, wherein Y is methylene or ethylene and at least one R is methyl or ethyl.

26. The process of claim 25, wherein

$$R—O—\overset{\overset{O}{\|}}{C}—(CH_2)_n—$$

group is in the ortho position on the phenyl ring with respect to the amine-containing side chain, and n is 0.

27. The process of claim 26, wherein Y is ethylene and R is ethyl.

28. The process of claim 22, 23, 24, 25, 26 or 27, wherein the product (a), (b), (c) or (d) is converted to the hydrochloride, sulfate, phosphate gluconate or tartrate acid addition salt.

**Patentansprüche:**

1. Eine Verbindung der Formel

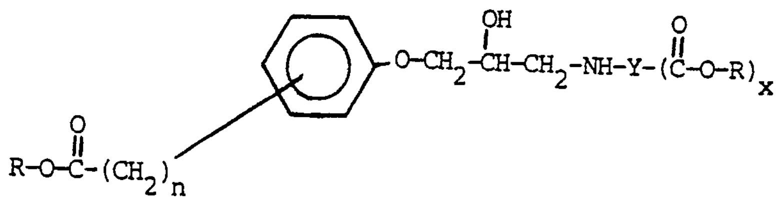 ,

worin Y eine geradkettige oder verzweigte divalente oder trivalente Kohlenwasserstoffgruppe mit 1—10 Kohlenstoffatomen oder Aralkyl mit 8—20 Kohlenstoffatomen jedes R niederes Alkyl mit 1—10 Kohlenstoffatomen, niederes Alkenyl mit 2—10 Kohlenstoffatomen, niederes Alkinyl mit 2—10 Kohlenstoffatomen, Aryl mit 6—10 Kohlenstoffatomen oder Aralkyl, worin der Alkylanteil 1—10 Kohlenstoffatome und der Arylanteil 6—10 Kohlenstoffatome enthält, bedeuten, die

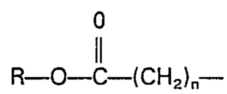

Gruppe bezogen auf die Amin-Seitenkette in ortho- oder para-Position am Phenylring ist, und n eine Zahl von 0—2 sowie x 1 oder 2 sind, oder ein pharmazeutisch verträgliches Salz der Verbindung.

2. Verbindung nach Anspruch 1, worin Y eine gerade oder verzweigte Kohlenstoffkette mit 1—6 Kohlenstoffatomen oder Aralkyl mit 8—12 Kohlenstoffatomen bedeutet und mindestens ein R niederes Alkyl mit 1—5 Kohlenstoffatomen, niederes Alkenyl mit 2—5 Kohlenstoffatomen, niederes Alkinyl mit 2—5 Kohlenstoffatomen ist, oder ein pharmazeutisch verträglisches Salz der Verbindung.

3. Verbindung nach Anspruch 1, worin Y eine gerade oder verzweigt Kohlenstoffkette mit 1—4 Kohlenstoffatomen bedeutet und mindestens ein R niederes Alkyl mit 1—4 Kohlenstoffatomen ist, oder ein pharmazeutisch verträgliches Salz der Verbindung.

4. Verbindung nach Anspruch 1, worin Y Methylen oder Ethylen bedeutet und mindestens ein R Methyl oder Ethyl ist, oder ein pharmazeutisch verträgliches Salz der Verbindung.

5. Verbindung nach Anspruch 4, worin die

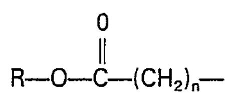

Gruppe bezogen auf die Amin-Seitenkette in ortho-Position am Phenylring ist und n 0 bedeutet.

6. Verbindung der Formel

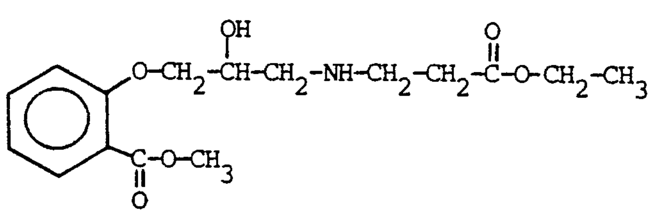

oder ein pharmazeutisch verträgliches Salz der Verbindung.

7. Verbindung nach Anspruch 1, 2, 3, 4, 5 oder 6 als das Hydrochlorid-, Sulfat-, Phosphat-, Gluconat- oder Tartrat-Säureadditionssalz.

8. Eine pharmazeutische Zussammensetzung, die eine wirksame Menge einer β-Blocker-Esterverbindung der Formel

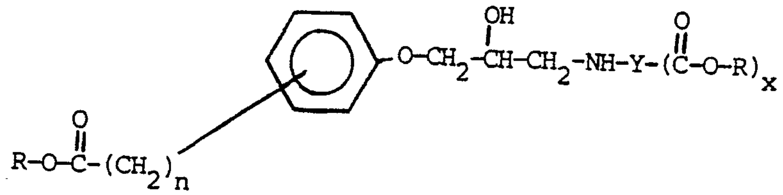

enthält oder daraus besteht, worin Y eine geradkettige oder verzweigte divalente oder trivalente Kohlenwasserstoffgruppe mit 1—10 Kohlenstoffatomen oder Aralkyl mit 8—20 Kohlenstoffatomen, jedes R niederes Alkyl mit 1—10 Kohlenstoffatomen, niederes Alkenyl mit 2—10 Kohlenstoffatomen, niederes Alkinyl mit 2—10 Kohlenstoffatomen, Aryl mit 6—10 Kohlenstoffatomen oder Aralkyl, worin der Alkylanteil 1—10 Kohlenstoffatome und der Arylanteil 6—10 Kohlenstoffatome enthält, bedeuten, die

$$R—O—\overset{\overset{O}{\|}}{C}—(CH_2)_n—$$

Gruppe bezogen auf die Amin-Seitenkette in ortho- oder para-Position am Phenylring ist, und n eine Zahl von 0—2 sowie x 1 oder 2 sind, oder ein pharmazeutisch verträgliches Salz der Verbindung, wobei die Verbindung in einer in geeigneter Weise gepufferten isotonischen Lösung gelöst geeignet zur intravenösen Anwendung ist.

9. Zusammensetzung nach Anspruch 8, worin Y eine gerade oder verzweigte Kohlenstoffkette mit 1—6 Kohlenstoffatomen oder Aralkyl mit 8—12 Kohlenstoffatomen bedeutet und mindestens ein R niederes Alkyl mit 1—5 Kohlenstoffatomen, niederes Alkenyl mit 2—5 Kohlenstoffatomen, niederes Alkinyl mit 2—5 Kohlenstoffatomen ist, oder ein pharmazeutisch verträgliches Salz der Verbindung.

10. Zusammensetzung nach Anspruch 8, worin Y eine gerade oder verzweigte Kohlenstoffkette mit 1—4 Kohlenstoffatomen bedeutet und mindestens ein R niederes Alkyl mit 1—4 Kohlenstoffatomen ist, oder ein pharmazeutisch verträgliches Salz der Verbindung.

11. Zusammensetzung nach Anspruch 8, worin Y Methylen oder Ethylen bedeutet und mindestens ein R Methyl oder Ethyl ist, oder ein pharmazeutisch verträgliches Salz der Verbindung.

12. Zusammensetsung nach Anspruch 11, worin die

$$R—O—\overset{\overset{O}{\|}}{C}—(CH_2)_n—$$

Gruppe bezogen auf die Amin-Seitenkette in ortho-Position am Phenylring ist und n 0 bedeutet.

13. Eine pharmazeutische Zusammensetzung, die eine wirksame Menge einer β-Blocker-Esterverbindung der Formel

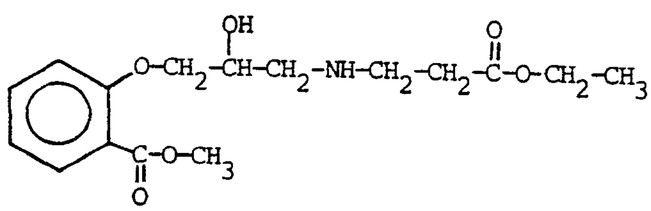

enthält oder daraus besteht oder eines pharmazeutisch verträglichen Salzes der Verbindung, wobei die Verbindung in einer in geeigneter Weise gepufferten isotonischen Lösung gelöst geeignet zur intravenösen Infusion ist.

14. Zusammensetzung nach Anspruch 8, 9, 10, 11, 12 oder 13, worin die β-Blocker-Verbindung ein Hydrochlorid-, Sulfat-, Phosphat-, Gluconat- oder Tartrat-Säureadditionssalz ist.

15. Eine Verbindung der Formel

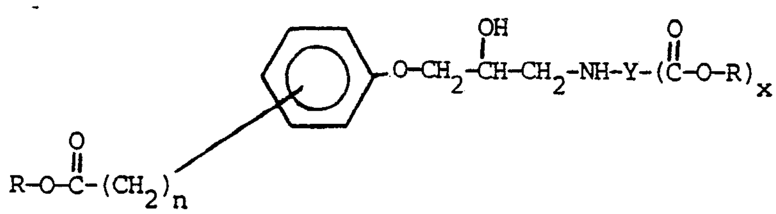

worin Y eine geradkettige oder verzweigte divalente oder trivalente Kohlenwasserstoffgruppe mit 1—10 Kohlenstoffatomen oder Aralkyl mit 8—20 Kohlenstoffatomen, jedes R niederes Alkyl mit 1—10 Kohlenstoffatomen, niederes Alkenyl mit 2—10 Kohlenstoffatomen, niederes Alkinyl mit 2—10 Kohlenstoffatomen, Aryl mit 6—10 Kohlenstoffatomen oder Aralkyl, worin der Alkylanteil 1—10 Kohlenstoffatome und der Arylanteil 6—10 Kohlenstoffatome enthält, bedeuten, die

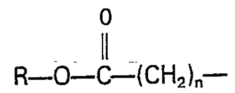

Gruppe bezogen auf die Amin-Seitenkette in ortho- oder para-Position am Phenylring ist, und n eine Zahl von 0—2 sowie x 1 oder 2 sind, oder ein pharmazeutisch verträgliches Salz der Verbindung zur Anwendung als ein β-Blocker-Verbindung.

16. Verbindung nach Anspruch 15, worin Y eine gerade oder verzweigte Kohlenstoffkette mit 1—6 Kohlenstoffatomen oder Aralkyl mit 8—12 Kohlenstoffatomen bedeutet und mindestens ein R niederes Alkyl mit 1—5 Kohlenstoffatomen, niederes Alkenyl mit 2—5 Kohlenstoffatomen, niederes Alkinyl mit 2—5 Kohlenstoffatomen ist, oder ein pharmazeutisch verträgliches Salz der Verbindung zur Anwendung als eine β-Blocker-Verbindung.

17. Verbindung nach Anspruch 15, worin Y eine gerade oder verzweigte Kohlenstoffkette mit 1—4 Kohlenstoffatomen bedeutet und mindestens ein R niederes Alkyl mit 1—4 Kohlenstoffatomen ist, oder ein pharmazeutisch verträgliches Salz der Verbindung zur Anwendung als eine β-Blocker-Verbindung.

18. Verbindung nach Anspruch 15, worin Y Methylen oder Ethylen bedeutet und mindestens ein R Methyl oder Ethyl ist, oder ein pharmazeutisch verträgliches Salz der Verbindung zur Anwendung als eine β-Blocker-Verbindung.

19. Verbindung nach Anspruch 18, worin die

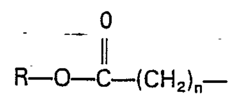

Gruppe bezogen auf die Amin-Seitenkette in ortho-Position am Phenylring ist und n 0 bedeutet zur Anwendung als eine β-Blocker-Verbindung.

20. Verbindung der Formel

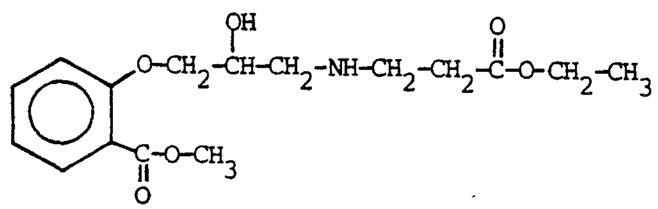

oder ein pharmazeutisch verträgliches Salz der Verbindung zur Anwendung als eine β-Blocker-Verbindung.

21. Verbindung nach Anspruch 15, 16, 17, 18, 19 oder 20 als das Hydrochlorid-, Sulfat-, Phosphat-, Gluconat- oder Tartrat-Säureadditionssalz zur Anwendung als eine β-Blocker-Verbindung.

22. Ein Verfahren zur Herstellung einer Verbindung der Formel

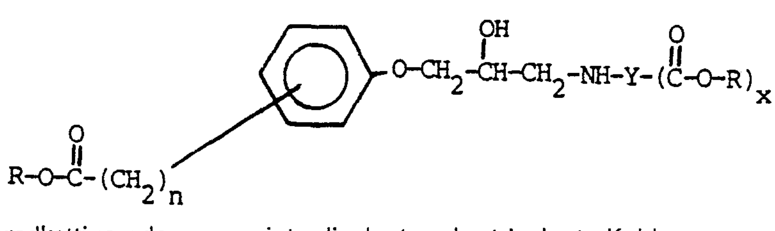

worin Y eine geradkettige oder verzweigte divalente oder trivalente Kohlenwasserstoffgruppe mit 1—10 Kohlenstoffatomen oder Aralkyl mit 8—20 Kohlenstoffatomen, jedes R ein niederes Alkyl mit 1—10 Kohlenstoffatomen, niederes Alkenyl mit 2—10 Kohlenstoffatomen, niederes Alkinyl mit 2—10 Kohlenstoffatomen, Aryl mit 6—10 Kohlenstoffatomen oder Aralkyl, worin der Alkylanteil 1—10 Kohlenstoffatome und der Aryl anteil 6—10 Kohlenstoffatome enthält, bedeuten, die

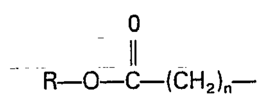

Gruppe bezogen auf die Amin-Seitenkette in ortho- oder para-Position am Phenylring ist, und n eine Zahl von 0—2 sowie x 1 oder 2 sind, oder ein pharmazeutisch verträgliches Salz der Verbindung, durch Umsetzung einer Arylhydroxy-Verbindung der Formel

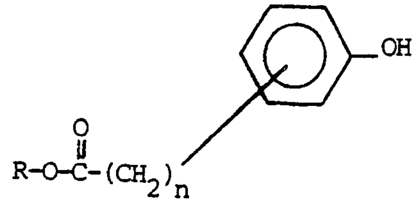

mit Epichlorhydrin in der Anwesenheit eines geeigneten Lösungsmittels und Base bei Rüchflußtemperatur unter Bildung eines Arylätherepoxids der Formel

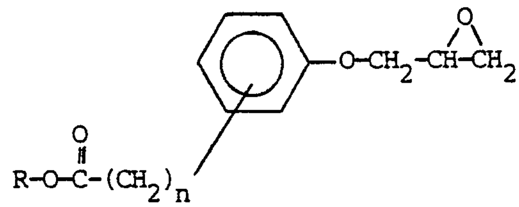

und anschließende Umsetzung des Arylätherepoxids gemäß einer der folgenden Verfahren unter Bildung des Endprodukts:

(a) das Arylätherepoxid wird mit einem geeigneten Amin der Formel $H_2N—Y—(COOR)_x$, worin Y und x die oben angegebene Bedeutung besitzen, in Gegenwart eines Lösungsmittels bei Rückflußtemperatur unter Bildung des Endproduktes;

(b) das Arylätherepoxid wird mit Succinimid und das sich ergebende Produkt mit einer geeigneten Säure unter Bildung eines Amins der Formel

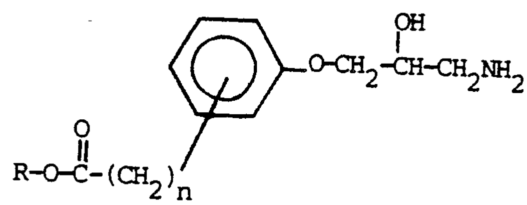

umgesetzt, welche sodann mit einer Verbindung der Formel Br—Y—COOR umgesetzt wird, worin Y und R die oben angegebene Bedeutung besitzen, unter Bildung des Endproduktes;

(c) das Arylätherepoxid wird mit einer Aminosäure der allgemeinen Formel $H_2N—Y—COOH$ umgesetzt, worin Y die oben angegebene Bedeutung besitzt, es folgt sodann die Umsetzung des Reaktionsprodukts mit einem Alkohol der Formel R—OH, worin R die oben angegebene Bedeutung besitzt, unter Bildung des Endproduktes;

(d) das Arylätherepoxid wird mit Benzylamin unter Erhitzung der Reaktionskomponenten zu dem Produkt der Formel

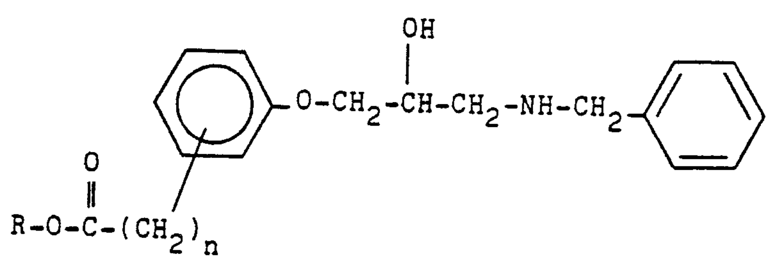,

welches sodann mit einer Verbindung der Formel

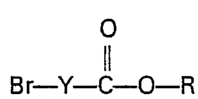

umgesetzt und das sich ergebende Produkt zur Entfernung der Benzylgruppe unter Bildung des Endproduktes hydriert wird; und gegebenenfalls Umsetzung des Endproduktes (a), (b), (c) oder (d) zu einem pharmazeutisch verträglichen Salz durch Umsetzung mit einer geeigneten Säure.

23. Verfahren nach Anspruch 22, worin Y eine gerade oder verzweigte Kohlenstoffkette mit 1—6 Kohlenstoffatomen oder Aralkyl mit 8—12 Kohlenstoffatomen bedeutet und mindestens ein R niederes Alkyl mit 1—5 Kohlenstoffatomen, niederes Alkenyl mit 2—5 Kohlenstoffatomen oder niederes Alkinyl mit 2—5 Kohlenstoffatomen ist.

24. Verfahren nach Anspruch 22, worin Y eine gerade oder verzweigte Kohlenstoffkette mit 1—4 Kohlenstoffatomen bedeutet und mindestens ein R niederes Alkyl mit 1—4 Kohlenstoffatomen ist.

25. Verfahren nach Anspruch 22, worin Y Methylen oder Ethylen bedeutet und mindesten sein R Methyl oder Ethyl ist.

26. Verfahren nach Anspruch 25, worin die

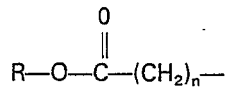

Gruppe bezogen auf die Amin-Seitenkette in ortho-Position am Phenylring ist und n 0 bedeutet.

27. Verfahren nach Anspruch 26, worin Y Ethylen und R Ethyl bedeuten.

28. Verfahren nach Anspruch 22, 23, 24, 25, 26 oder 27, worin das Produkt (a), (b), (c) oder (d), zu dem Hydrochlorid-, Sulfat-, Phosphat-, Gluconat- oder Tartrat-Säureadditionssalz umgewandelt wird.

**Revendications**

1. Un composé de formule

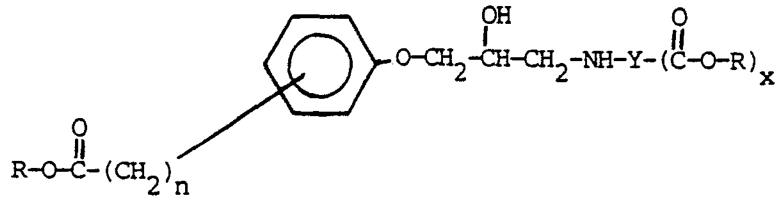

dans laquelle:

Y est un groupe hydrocarboné divalent ou trivalent à chaîne linéaire ou ramifiée, comprenant de 1 à 10 atomes de carbone, ou un radical aralkyle comprenant de 8 à 20 atomes de carbone; chaque groupe R est un radical alkyle infèrieur comprenant de 1 à 10 atomes de carbone, un radical alkényle inférieur comprenant de 2 à 10 atomes de carbone, un radical alkynyle inférieur comprenant de 2 à 10 atomes de carbone, un radical aryle comprenent de 6 à 10 atomes de carbone ou un radical aralkyle, dans lequel la partie alkyle contient de 1 à 10 atomes de carbone et la partie aryle contient de 6 à 10 atomes de carbone; le groupe

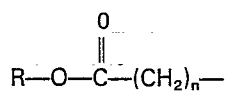

est en position ortho ou para sur le noyau phényle par rapport à la chaîne latérale contenant l'amine; n est un nombre entier compris entre 0 et 2; et x est égal à 1 ou 2, ou un de leurs sels pharmaceutiquement acceptables.

2. Le composé selon la revendication 1, dans lequel Y est une chaîne carbonée linéaire ou ramifiée comprenant de 1 à 6 atomes de carbone, ou un radical aralkyle comprenant de 8 à 12 atomes de carbone; et au moins un groupe R est un radical alkyle inférieur comprenant de 1 à 5 atomes de carbone, un radical alkényle inférieur comprenant de 2 à 5 atomes de carbone, un radical alkynyle inférieur comprenant de 2 à 5 atomes de carbone; ou un de ses sels pharmaceutiquement acceptables.

3. Le composé selon la revendication 1, dans lequel Y est une chaîne hydrocarbonée linéaire ou ramifiée comprenant de 1 à 4 atomes de carbone et au moins un des groupes R est un alkyle inférieur comprenant de 1 à 4 atomes de carbone; ou un de ses sels pharmaceutiquement acceptables.

4. Le composé selon la revendication 1, dans lequel Y est un radical méthylène au éthylène et au moins un des groupes R est un radical méthyle ou éthyle; ou un de sels pharmaceutiquement acceptables en dérivant.

5. Le composé selon la revendication 4, dans lequel le groupe R—O—C(O)—$(CH_2)_n$— est en position ortho sur le noyau phényle par rapport à la chaîne latérale contenant l'amine, et n est égal à 0.

6. Le composé présentant la formule:

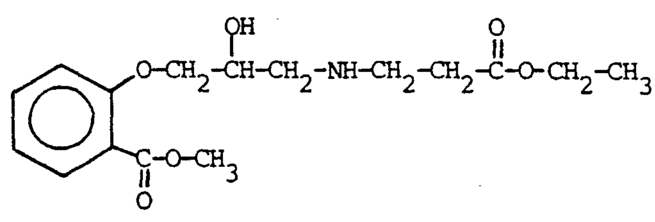

ou un de ses sel pharmaceutiquement acceptables.

7. Le composé selon la revendication 1, 2, 3, 4, 5 ou 6 sous la forme de son sel d'addition acide avec l'un des acides chlorhydrique, sulfurique, phosphorique, gluconique ou tartrique.

8. Une composition pharmaceutique comprenant la quantité necessaire d'un agent β-bloquant consistant en un ester β-bloquant présentant la formule:

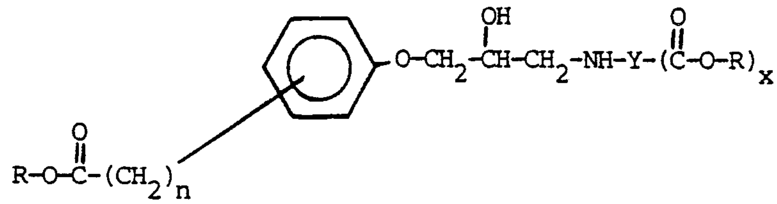

dans laquelle:

Y est un groupe hydrocarboné divalent ou trivalent à chaîne linéaire ou ramifiée, comprenant de 1 à 10 atomes de carbone, ou un radical aralkyle comprenant de 8 à 20 atomes de carbone; chaque groupe R est un radical alkyle inférieur comprenant de 1 à 10 atomes de carbone, un radical alkényle inférieur comprenant de 2 à 10 atomes de carbone, un radical alkynyle inférieur comprenant de 2 à 10 atomes de carbone, un radical aryle comprenant de 6 à 10 atomes de carbone ou un radical aralkyle, dans lequel la partie alkyle contient de 1 à 10 atomes de carbone et la partie aryle contient de 6 à 10 atomes de carbone; le groupe

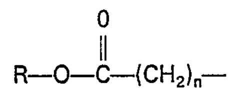

est en position ortho ou para sur le noyau phényle par rapport à la chaîne latérale contenant l'amine; n est un nombre entier compris entre 0 et 2; et x est égal à 1 ou 2, ou un de leurs sels pharmaceutiquement acceptables, ce composé étant dissous dans une solution isotonique convenablement tamponnée pour l'administration intraveineuse.

9. La composition selon la revendication 8, dans laquelle Y est une chaîne hydrocarbonée linéaire ou ramifiée comprenant de 1 à 6 atomes de carbone, ou un radical aralkyle comprenant de 8 à 12 atomes de carbone et dont au moins un des groupes R est un radical alkyle inférieur comprenant de 1 à 5 atomes de carbone, un radical alkényle inférieur comprenant de 2 à 5 atomes de carbone, un radical alkynyle inférieur comprenant de 2 à 5 atomes de carbone; ou un sel pharmaceutiquement acceptable en dérivant.

10. La composition selon la revendication 8, dans laquelle Y est un chaîne hydrocarbonée linéaire ou ramifiée comprenant de 1 à 4 atomes de carbone, au moins un des groupes R étant un radical alkyle inférieur comprenant de 1 à 4 atomes de carbone; ou un sel pharmaceutiquement acceptable.

11. La composition selon la revendication 8, dans laquelle Y est un radical méthylène ou éthylène et au moins un groupe R est un radical méthyle ou éthyle; ou un sel pharmaceutiquement acceptable en dérivant.

12. La composition selon la revendication 11, dans laquelle le groupe $R—O—C(O)—(CH_2)_n—$ est en position ortho sur le noyau phényl par rapport à la chaîne latérale contenant l'amine, et n est égal à 0.

13. Une composition pharmaceutique comprenant la quantité nécessaire d'un agent β-bloquant consistant en un composé ester de β-bloquant présentant la formule:

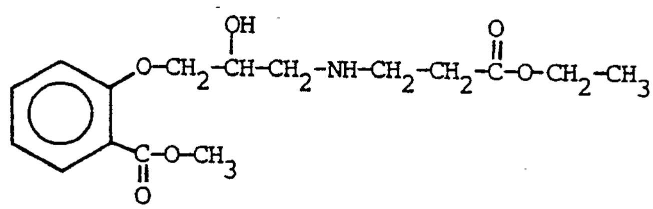

ou un de ses sels pharmaceutiquement acceptables, ce composé étant dissous dans une solution isotonique convenablement tamponnée pour l'administration intraveineuse.

14. La composition selon la revendication 8, 9, 10, 11, 12 ou 13, dans laquelle le composé β-bloquant est un sel d'addition acide avec l'un des acides chlorhydrique, sulfurique, phosphorique, gluconique ou tartrique.

15. Un composé présentant la formule:

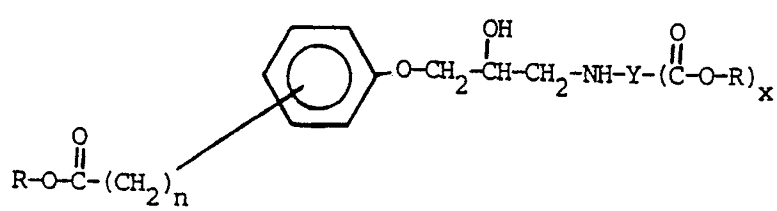

dans laquelle:

Y est un groupe hydrocarboné divalent ou trivalent à chaîne linéaire ou ramifiée, comprenant de 1 à 10 atomes de carbone, ou un radical aralkyle comprenant de 8 à 20 atomes de carbone; chaque groupe R est un radical alkyle inférieur comprenant de 1 à 10 atomes de carbone, un radical alkényle inférieur comprenant de 2 à 10 atomes de carbone, un radical alkynyle inférieur comprenant de 2 à 10 atomes de carbone, un radical aryle comprenant de 6 à 10 atomes de carbone ou un radical aralkyle, dans lequel la partie alkyle contient de 1 à 10 atomes de carbone et la partie aryle contient de 6 à 10 atomes de carbone; le groupe

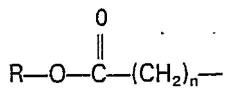

est en position ortho ou para sur le noyau phényle par rapport à la chaîne latérale contenant l'amine; n est un nombre entier compris entre 0 et 2; et x est égal à 1 ou 2, ou leurs sels pharmaceutiquement acceptables destinés à être utilisés en tant qu'agents β-bloquants.

16. Le composé selon la revendication 15, dans laquel Y est une chaîne hydrocarbonée linéaire ou ramifiée comprenant de 1 à 6 atomes de carbone, ou un radical aralkyle comprenant de 8 à 12 atomes de carbone; au moins un des groupes R est un radical alkyle inférieur comprenant de 1 à 5 atomes de carbone, un radical alkényle inférieur comprenant de 2 à 5 atomes de carbone, un radical alkynyle inférieur comprenant de 2 à 5 atomes de carbone; ou leurs sels pharmaceutiquement acceptables, destinés à être utilisés en tant qu'agent β-bloquant.

17. Le composé selon la revendication 15, dans lequel Y est une chaîne hydrocarbonée linéaire ou ramifiée comprenant de 1 à 4 atomes de carbone, au moins un des groupes R est un radical alkyle inférieur comprenant de 1 à 4 atomes de carbone; ou leurs sels pharmaceutiquement acceptables, destinés à être utilisés en tant qu'agents β-bloquants.

18. Le composé selon la revendication 15, dans lequel Y est un radical méthylène ou èthylène et R est un radical méthyle ou èthyle, ou leurs sels pharmacetiquement acceptable, destinés à être utilisés en tant qu'agent β-bloquant.

19. Le composé selon la revendication 18, dans lequel le groupe $R—O—C(O)—(CH_2)_n—$ est en position ortho sur le noyau phényle par rapport à la chaîne latérale contenant l'amine et n est égal à 0, destiné à être utilisé en tant qu'agent β-bloquant.

20. Le composé présentant la formule:

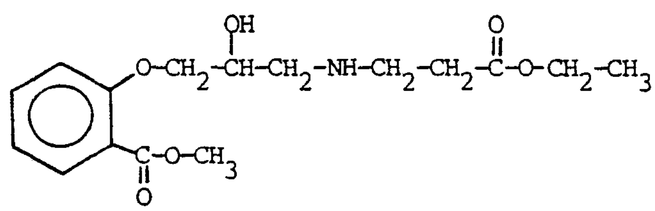

ou ses sels pharmaceutiquement acceptables, destinés à être utilisés en tant qu'agent β-bloquant.

21. Le composé selon la revendication 15, 16, 17, 18, 19 ou 20 sous la forme de son sel d'addition acide avec l'un des acides chlorhydrique, sulfurique, phosphorique, gluconique ou tartrique, destiné à être utilisé en tant qu'agent β-bloquant.

22. Un procédé de préparation d'un composé de formule:

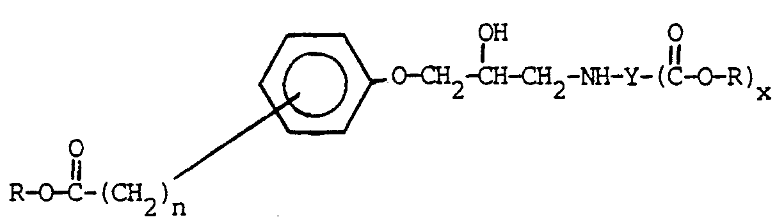

dans laquelle:

Y est un groupe hydrocarboné divalent ou trivalent à chaîne linéaire ou ramifiée, comprenant de 1 à 10 atomes de carbone, ou un radical aralkyle comprenant de 8 à 20 atomes de carbone; chaque groupe R est un radical alkyle inférieur comprenant de 1 à 10 atomes de carbone, un radical alkényle inférieur comprenant de 2 à 10 atomes de carbone, un radical alkynyle infèrieur comprenant de 2 à 10 atomes de carbone, un radical aryle comprenant de 6 à 10 atomes de carbone ou un radical aralkyle, dans lequel la

partie alkyle contient de 1 à 10 atomes de carbone et la partie aryle contient de 6 à 10 atomes de carbone; le groupe

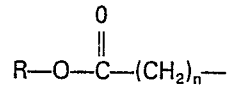

est en position ortho ou para sur le noyau phényle par rapport à la chaîne latérale contenant l'amine; n est un nombre entier compris entre 0 et 2; et x est égal à 1 ou 2, ou leurs sels pharmaceutiquement acceptables, ce procédé consistant à faire réagir un composé aryl-hydroxy de formule:

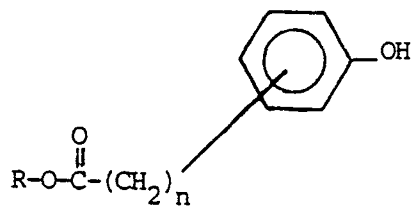

avec une épichlorohydrine en présence d'un solvant convenable et d'une base, à température du reflux pour conduire à un aryl-éther-époxyde de formule:

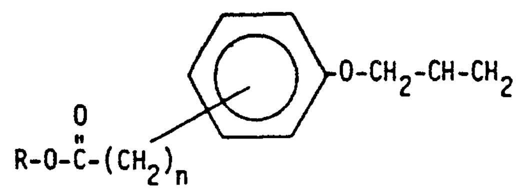

suivie d'une réaction de l'aryl-éther-époxyde conformément à l'une ou l'autre des méthodes suivantes, de façon à conduire au produit final:

(a) l'aryl-éther-époxyde réagit avec une amine convenable de formule $H_2N—Y—(COOR)_x$ dans laquelle Y et x sont tels que définis ci-dessus, en présence d'un solvant à la température du reflux pour conduire au produit final;

(b) l'aryl-éther-époxyde réagit avec le succinimide puis le produit résultant réagit avec un acide convenable pour conduire à une amine de formule:

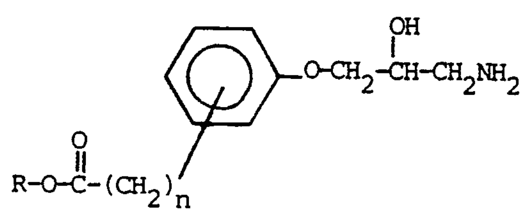

qui agit à son tour avec un composé de formule Br—Y—COOR, dans lequel Y et R sont tels que définis ci-dessus, pour conduire au produit final;

(c) l'aryl-éther-époxyde réagit avec un aminoacide de formule $H_2N—Y—COOH$, dans laquelle Y est tel que défini ci-dessus, puis le produit obtenu réagit avec un alcool de formule R—OH dans laquelle R est tel que dèfini ci-dessus, pour conduire au produit final;

(d) l'aryl-éther-époxyde réagit avec la benzylamine par chauffage des réactifs pour conduire au produit de formule:

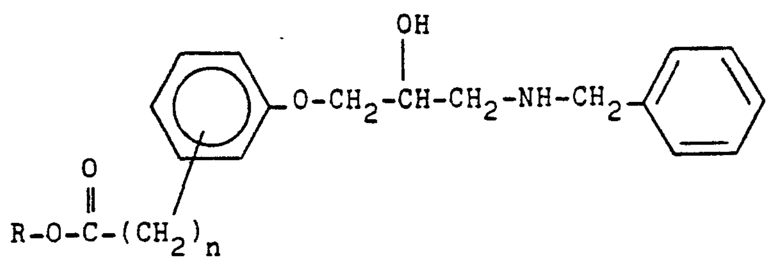

qui réagit alors avec un composé de formule Br—Y—C(O)—O—R; le produit résultant étant hydrogéné

pour éliminer le groupe benzyle conduit au produit final; et éventuellement convertir le produit final (a), (b), (c) ou (d) en un sel pharmaceutiquement acceptable par réaction avec un acide convenable.

23. Le procédé selon la revendication 22, dans lequel Y est une chaîne hydrocarbonée linéaire ou ramifiée comprenant de 1 à 6 atomes de carbone ou un radical aralkyle comprenant de 8 à 12 atomes de carbone, et au moins un des groupes R est un radical alkyle inférieur comprenant de 1 à environ 5 atomes de carbone, un radical alkényle inférieur comprenant de 2 à environ 5 atomes de carbone ou un radical alkynyle inférieur comprenant de 2 à 5 atomes de carbone.

24. Le procédé selon la revendication 22, dans lequel Y est un hydrocarbure à chaîne linéaire ou ramifiée comprenant de 1 à 4 atomes de carbone, et au moins un des groupes R est un radical alkyle inférieure comprenant 1 à 4 atomes de carbone.

25. Le procédé selon la revendication 22, dans lequel Y est un radical méthylène ou éthylène et au moins un des groupes R est un radical méthyle ou éthyle.

26. Le procédé selon la revendiction 25, dans lequel le groupe $R—O—C(C)—(CH_2)_n—$ est en position ortho sur le noyau phényle par rapport à la chaîne latérale contenant l'amine et n est égal à 0.

27. Le procédé selon la revendication 26, dans lequel Y est l'éthylène et R est un radical èthyle.

28. Le procédé selon la revendication 22, 23, 25, 26 ou 27, dans lequel le produit (a), (b), (c) ou (d) est converti en un sel d'addition d'acide chlorhydrique, sulfurique, phosphorique, gluconique ou tartrique.